# EUROPÄISCHE PATENTSCHRIFT

(11) **EP 1 569 898 B1**
(45) Veröffentlichungstag und Bekanntmachung des Hinweises auf die Patenterteilung: **07.01.2015**
(21) Anmeldenummer: 03785687.9
(22) Anmeldetag: 28.11.2003
(51) Int. Cl.: C07D 203/08, C07D 487/08

(54) **STABILISIERTE ZUSAMMENSETZUNGEN, ENTHALTEND POLYFUNKTIONELLE AZIRIDINVERBINDUNGEN ALS HÄRTERKOMPONENTE**
STABILISED COMPOSITIONS CONTAINING POLYFUNCTIONAL AZIRIDINE COMPOUNDS AS HARDENING CONSTITUENTS
COMPOSITIONS STABILISÉES CONTENANT DES COMPOSÉS AZIRIDINE POLYFONCTIONNELS UTILISÉS COMME COMPOSANTS DURCISSEURS

(30) Priorität: 03.12.2002 DE 10256494
(43) Veröffentlichungstag der Anmeldung: 07.09.2005
(73) Patentinhaber: BASF SE, 67056 Ludwigshafen (DE)
(72) Erfinder: DECKER, Jürgen, 67346 Speyer (DE); ADAMS, Stefan, 67065 Ludwigshafen (DE)
(86) Internationale Anmeldenummer: PCT/EP2003/013424
(87) Internationale Veröffentlichungsnummer: WO 2004/050617

(56) Entgegenhaltungen:
- US-A- 4 025 503
- US-A1- 2003 208 033
- DATABASE CA [Online] CHEMICAL ABSTRACTS SERVICE, COLUMBUS, OHIO, US; 3. Juni 1985 (1985-06-03) NIPPON POLYURETHANE INDUSTRY: "Molding of polyisocyanurate heat-resistant resins" Database accession no. 102:185972 XP002274798 & JP 59 221321 A (NIPPON POLYURETHANE INDUSTRY CO. LTD, JAPAN) 12. Dezember 1984 (1984-12-12) & EP 0 469 459 A (TEIJIN LIMITED, JAPAN) 5. Februar 1992 (1992-02-05)
- DATABASE CA [Online] CHEMICAL ABSTRACTS SERVICE, COLUMBUS, OHIO, US; 26. Juli 1982 (1982-07-26) MITSUBISHI PAPER MILLS LTD, JAPAN: "Hardening of gelatins during preparation of silver halide photographic materials by simultaneous coating method" Database accession no. 97:31223 XP002274799 & JP 51 009434 A (MITSUBISHI PAPER MILLS LTD, JAPAN) 26. Januar 1976 (1976-01-26)

## Beschreibung

Die vorliegende Erfindung betrifft ein Verfahren zur Herstellung einer Zusammensetzung, die mindestens eine polyfunktionelle Aziridinverbindung und 1,4-Diazabicyclo[2.2.2]octan enthält. Weiterer Gegenstand der vorliegenden Erfindung ist die Verwendung dieser Zusammensetzung als Härterkomponente.

Aziridinverbindungen sind bereits seit langem bekannt und werden vielfach als Härterkomponenten z. B. für Formulierungen im Bereich der Lederbehandlung verwendet. Aziridine sind infolge ihrer Ringspannung reaktive Verbindungen, die mit einer Vielzahl an Reagenzien unter Ringöffnung reagieren. Ein Nachteil dieser hohen Reaktivität der Aziridineinheit ist die nur eingeschränkte Lagerfähigkeit der Aziridinverbindungen, da die Produkte mit sich selbst reagieren und verdicken können. Eine solche Verdickung setzt die Wirksamkeit von Aziridinverbindungen als Härterkomponenten herab, da einerseits die Anzahl der aktiven Aziridineinheiten sinkt und andererseits die Löslichkeit und die Verdünnbarkeit der Härterkomponente abnimmt. Im Extremfall führt eine Verdickung zu einem völlig unlöslichen und damit unbrauchbaren Härterprodukt. Im Stand der Technik sind unterschiedliche Verfahren zur Stabilisierung von Aziridinverbindungen beschrieben.

Die US 3,671,256 beschreibt die Verwendung von Aziridineinheiten in der Seitenkette von Polymerhärtern, wobei die Aziridineinheiten durch Verdünnung in geeigneten, nicht reaktiven Lösemitteln stabilisiert werden.

Die US 4,960,687 beschreibt ein Verfahren zur Herstellung einer Trägerschicht für einen photographischen Film. In diesem Verfahren wird unter anderem eine aziridinhaltige Verbindung als Härterkomponente verwendet. Aufgrund der geringen Stabilität dieser Aziri dinverbindung muss sie durch die Einstellung eines pH-Wertes zwischen 9,0 und 11,5 in einem wässrigen System stabilisiert werden.

JP 59221321 offenbart ein Verfahren zur Verformung von Hitze-resistenten Polyisocyanurat-basierten Harzen, worin zwei Komponenten verwendet werden. Die erste Komponente umfasst eine flüssige Mischung eines Präpolymers, welches organische Polyisocyanate und/oder Isocyanat-Gruppen und eine Epoxyverbindung enthält. Die zweite Komponente umfasst eine flüssige Mischung einer Aziridinverbindung und eines tertiären Amins, und gegebenenfalls einer Verbindung, die aktive Wasserstoffe enthält und/oder einer Verbindung, welche inerte Wasserstoffe aufweist.

JP 51009434 offenbart ein Verfahren zur Beschleunigung des Aushärtvorgangs eines Films, wobei eine Gelatine-enthaltende wässrige Zusammensetzung mit einem organischen Härter ausgewählt aus der Gruppe bestehend aus (1) Härtern mit aktiven Halogenen, (2) Härtern des Olefintyps, (3) Härtern des Epoxidtyps und (4) Härtern des Aziridintyps eingesetzt wird. Zusätzlich zu den genannten Härtern enthält die Zusammensetzung gemäß diesem Dokument wasserlösliche Alkalien, Alkalisalze und primäre bis tertiäre Amine.

US 4,025,503 betrifft ein Verfahren zur Umesterung von Methyl-, Ethyl- oder Propylaziridinestern mit höherwertigen Alkoholen, beispielsweise die Umesterung von Aziridinpropionsäureester mit Polyethylenglykolether. Die Umesterung wird dabei in Gegenwart von Paraffinkohlenwasserstoffen und einem tertiären Amin, beispielsweise DABCO, als Katalysator durchgeführt.

Nachteilig an diesen Stabilisierungsmethoden von Aziridinverbindungen ist, dass der Anwender bei der Verwendung von aziridinhaltigen Verbindungen entweder an ein bestimmtes Lösemittel oder aber an die Verwendung von wässrigen Lösungen mit bestimmten pH-Werten gebunden ist. Insbesondere die Verwendung von wässrigen Lösungen von aziridinhaltigen Zusammensetzungen kann zu Schwierigkeiten führen, da manche aziridinhaltige Verbindung in Gegenwart von Wasser nur eine begrenzte Haltbarkeit aufweist.

Die Aufgabe der vorliegenden Erfindung besteht somit darin, eine nicht-wässrige aziridinhaltige Zusammensetzung bereitzustellen, in der die Aziridineinheit ohne die in Rede stehenden Nachteile stabilisiert wird. Ferner soll ein Verfahren zur Herstellung dieser aziridinhaltigen Zusammensetzung bereitgestellt werden.

Es wurde erfindungsgemäß gefunden, dass 1,4-Diazabicyclo[2.2.2]octan stabilisierend auf polyfunktionelle Aziridinverbindungen wirkt.

Gegenstand der vorliegenden Erfindung ist somit ein Verfahren zur Herstellung einer Zusammensetzung enthaltend mindestens eine polyfunktionelle Aziridinverbindung, 1,4-Diazabicyclo[2.2.2]octan und ein polares, nicht reaktives Lösemittel, ausgewählt aus Diacetonalkohol oder N-Methyl-pyrrolidinon, wobei der Gehalt an 1,4-Diazabicyclo[2.2.2]octan 0,1 bis 10 Gew.-%, jeweils bezogen auf die Zusammensetzung, beträgt, gekennzeichnet durch die folgenden Verfahrensschritte:
(a) Bereitstellung einer Mischung aus gegebenenfalls substituiertem Ethylenimin und 1,4-Diazabicyclo[2.2.2]octan;
(b) Zugabe mindestens eines mit α,β-ungesättigten Carbonsäuren veresterten mehrwertigen Alkohols und/oder mindestens eines Polyisocyanates,
die Verwendung einer Zusammensetzung, enthaltend mindestens eine polyfunktionelle Aziridinverbindung, 1,4-Diazabicyclo[2.2.2]octan und ein polares, nicht reaktives Lösemittel, ausgewählt aus Diacetonalkohol oder N-Methyl-pyrrolidinon, mit einem Gehalt an l,4-Diazabicyclo[2.2.2]octan von 0,1 bis 10 Gew.-%, jeweils bezogen auf die Zusammensetzung, als Härterkomponente für Formulierungen im Bereich der Lederbehandlung, der Beschichtungen, des Textildrucks und der Lacke, sowie Lederbehandlungsmittel, Beschichtungsmittel, Textildruckmittel oder Lacke, enthaltend eine Zusammensetzung, enthaltend mindestens eine polyfunktionelle Aziridinverbindung, 1,4-Diazabicyclo[2.2.2]octan und ein polares, nicht reaktives Lösemittel, ausgewählt aus Diacetonalkohol oder N-Methyl-pyrrolidinon, mit einem Gehalt an 1,4-Diazabicyclo[2.2.2]octan von 0,1 bis 10 Gew.-%, jeweils bezogen auf die Zusammensetzung als Härterkomponente.

1,4-Diazabicyclo[2.2.2]octan (I), DABCO genannt, ist ein bicyclisches Triethylendiamin, das durch Erhitzen von N-Hydroxyethylpiperazin hergestellt werden kann, und hauptsächlich als Katalysator bei der Polyurethan-Verschäumung und bei Veresterungen verwendet wird.

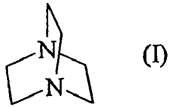

Die erfindungsgemäße Zusammensetzung enthält mindestens eine polyfunktionelle Aziridinverbindung mit mindestens zwei Struktureinheiten der allgemeinen Formel (II), 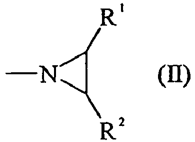 wobei R¹ und R², unabhängig voneinander, jeweils ein Wasserstoffatom oder einen gegebenenfalls funktionalisierten Alkyl-, Alkenyl-, Aryl- oder Aralkylrest bedeuten.

Der Gehalt an 1,4-Diazabicyclo[2.2.2]octan in der erfindungsgemäßen Zusammensetzung beträgt 0,1 bis 10 Gew.-%, insbesondere 0,3 bis 5 Gew.-%, bevorzugt 0,5 bis 2,5 Gew.-%, jeweils bezogen auf die Zusammensetzung.

Die polyfunktionelle Aziridinverbindung, die in der erfindungsgemäßen Zusammensetzung enthalten ist, kann vorzugsweise ausgewählt werden aus der Gruppe bestehend aus den Michael-Additionsprodukten von gegebenenfalls substituiertem Ethylenimin an Ester von mehrwertigen Alkoholen mit α,β-ungesättigten Carbonsäuren und den Additionsprodukten von gegebenenfalls substituiertem Ethylenimin an Polyisocyanaten.

Geeignete Alkoholkomponenten sind beispielsweise Trimethylolpropan, Neopentylglykol, Glycerin, Pentaerythrit, 4,4'-Isopropylidendiphenol und 4,4'-Methylendiphenol. Als α,β-ungesättigte Carbonsäuren kommen beispielsweise Acryl- und Methacrylsäure, Crotonsäure und Zimtsäure in Frage.

Besonders bevorzugt enthält die erfindungsgemäße Zusammensetzung Acrylsäureester.

Die korrespondierenden mehrwertigen Alkohole der α,β-ungesättigten Carbonsäureester können gegebenenfalls Alkohole sein, die an ihren OH-Funktionen teilweise oder vollständig mit Alkylenoxiden einfach oder mehrfach verlängert sind. Hierbei kann es sich beispielsweise um die mit Alkylenoxiden einfach oder mehrfach verlängerten oben genannten Alkohole handeln. Diesbezüglich wird auch auf die US 4,605,698 verwiesen Erfindungsgemäß besonders geeignete Alkylenoxide sind Ethylenoxid und Propylenoxid.

Beispiele für erfindungsgemäß geeignete Aziridine sind Trimethylolpropantris-(beta-aziridino)-propionat, Neopentylglykoldi-(beta-aziridino)-propionat, Glycerintris-(beta-aziridino)-propionat, Pentaerythrittetra-(beta-aziridino)-propionat, 4,4'-Isopropylidendiphenoldi-(beta-aziridino)-propionat, 4,4'-Methylendiphenoldi-(beta-aziridino)-propionat, 1,6-Hexamethylen-di-(N,N-ethylenharnstoff), 4,4'-Methylen-bis-(phenyl-N,N-ethylenharnstoff), 1,3,5-Tris-(ω-hexamethylen-N,N-ethylenharnstoff)-biuret und Gemische davon. Die polyfunktionellen Aziridinverbindungen können gegebenenfalls an ihren Aziridineinheiten substituiert sein.

Die erfindungsgemäße Zusammensetzung umfasst zusätzlich ein polares, nicht reaktives Lösemittel, das eines oder mehrere der folgenden Merkmale aufweisen kann: organisch, wässrig mischbar, nicht toxisch und preiswert. Erfindungsgemäß liegt als polares, nicht reaktives Lösemittel Diacetonalkohol oder N-Methylpyrrolidinon vor. Der Gehalt an Lösemittel in der erfindungsgemäßen Zusammensetzung beträgt vorzugsweise 1 bis 50 Gew.-%, besonders bevorzugt 2 bis 40 Gew.-%, insbesondere 3 bis 30 Gew.-%, speziell 4 bis 20 Gew.-%, jeweils bezogen auf die Zusammensetzung.

Der in b) zugegebene Ester ist ein mehrfach mit α,β-ungesättigten Carbonsäuren veresterter Alkohol. Der Alkohol wird vorzugsweise ausgewählt aus der Gruppe bestehend aus Trimethylolpropan, Neopentylglykol, Glycerin, Pentaerythrit, 4,4'-Isopropylidendiphenol und 4,4'-Methylendiphenol. Als α,β-ungesättigten Carbonsäuren kommen beispielsweise Acryl- und Methacrylsäure, Crotonsäure und Zimtsäure in Frage.

Besonders bevorzugt werden Acrylsäureester in der erfindungsgemäßen Zusammensetzung verwendet.

Der Alkohol kann gegebenenfalls an seinen OH-Funktionen teilweise oder vollständig mit Alkylenoxideinheiten einfach oder mehrfach verlängert sein. Erfindungsgemäß besonders geeignet sind hierfür Ethylenoxid und Propylenoxid.

Das Polyisocyanat wird vorzugsweise ausgewählt aus der Gruppe bestehend aus Hexamethylendiisocyanat, 4,4'-Methylen-bis-(phenylisocyanat) und 1,3,5-Tris(ω-hexamethyleniso-cyanato)-biuret.

Ferner betrifft die vorliegenden Erfindung die Verwendung der erfindungsgemäßen Zusammensetzung als Härterkomponente für Formulierungen im Bereich der Lederbehandlung, der Beschichtungen, des Textildrucks und der Lacke, wobei die Lacke insbesondere im Bereich der Straßenmarkierung und der Anstrichfarben verwendet werden können. Die erfindungsgemäßen Zusammensetzungen erhöhen dabei die Wasserfestigkeit der Lacke.

Ferner betrifft die vorliegende Erfindung Lederbehandlungsmittel, Beschichtungsmittel, Textildruckmittel oder Lacke, welche die erfindungsgemäße Zusammensetzung als Härterkomponente enthalten. Dabei dienen die Lacke, die die erfindungsgemäßen Zusammensetzungen enthalten, vorzugsweise zur Straßenmarkierung oder als Anstrichfarben.

Die Lederbehandlungsmittel sind vorzugsweise wässrig.

Der Anteil an erfindungsgemäßer Zusammensetzung in den Lederbehandlungsmitteln beträgt vorzugsweise 0 bis 5 Gew.-%, besonders bevorzugt 0,1 bis 4 Gew.-%, insbesondere 0,2 bis 3 Gew.-%, speziell 0,3 bis 2 Gew.-%, jeweils bezogen auf das Lederbehandlungsmittel.

Die Lederbehandlungsmittel können gegebenenfalls weitere, für Lederrezepturen übliche Zusatzstoffe aufweisen. Beispiele hierfür sind Farbpigmente, Füllmittel, Bindemittel, Grundierungen, (Matt-)Appreturen, Wachse, Griffmittel, Entschäumer, Verlaufsmittel und Farbstoffe.

Der Anteil an Zusatzstoffen in diesen Lederbehandlungsmitteln beträgt vorzugsweise 0 bis 75 Gew.-%, besonders bevorzugt 10 bis 65 Gew.-%, insbesondere 20 bis 60 Gew.-%, speziell 25 bis 55 Gew.-%, jeweils bezogen auf das Lederbehandlungsmittel.

Die vorliegende Erfindung zeigt eine Reihe von Vorteilen gegenüber dem Stand der Technik.

Erfindungsgemäß erfolgt die Stabilisierung der polyfunktionellen Aziridinverbindungen durch 1,4-Diazabicyclo[2.2.2]octan. Eine Einstellung eines bestimmten pH-Wertes - was gegebenenfalls unvereinbar mit bestimmten Anwendungen der polyfunktionellen Aziridinverbindungen ist - ist nicht notwendig. Die resultierenden erfindungsgemäßen Zusammensetzungen sind lagerstabil, hellgelb und lassen sich sehr gut verarbeiten. Sie sind gut mit Wasser verdünnbar und weisen eine relativ lange Verarbeitungszeit auf.

Die vorliegende Erfindung wird anhand der folgenden Ausführungsbeispiele näher erläutert.

### Ausführungsbeispiele:

Alle Mengenangaben in den Ausführungsbeispielen sind in Gewichtsteilen angegeben.

### a) Viskosität und Wasserverdünnbarkeit

Die erfindungsgemäßen Formulierungen werden durch Reaktion von Ethylenimin und Trimethylolpropantrisacrylat hergestellt. Dabei wird 1,4-Diazabicyclo[2.2.2]octan vor der Synthese der Aziridinverbindung zu dem Ethylenimin gegeben. Erst danach erfolgt die Zugabe des Acrylats.

Die Viskosität der Proben wurden nach Brookfield bei 23 °C gemessen. Die Wasserverdünnbarkeit - Homogenität, Verarbeitbarkeit - wurde mit Schulnoten beurteilt.

Folgende Formulierungen wurden hergestellt

| | |
|---|---|
| A1 (erfindungsgemäß): | 90,2 Teile (III), 8,2 Teile N-Methylpyrrolidon (NMP), 1,6 Teile 1,4-Diazabicyclo[2.2.2]octan |
| A2 (erfindungsgemäß): | 90,2 Teile (III), 8,2 Teile Diacetonalkohol, 1,6 Teile 1,4-Diazabicyclo[2.2.2]octan |
| B (Vergleichsbeispiel): | 100 Teile (III) |
| C (Vergleichsbeispiel): | 91,8 Teile (III), 8,2 Teile Diacetonalkohol |
| D (Vergleichsbeispiel): | 98,4 Teile (III), 1,6 Teile 1,4-Diazabicyclo[2.2.2]octan |
| E (Stand der Technik): | 99,0 Teile (III), 1,0 Teile Tetramethylethylendiamin (TMEDA) |

mit

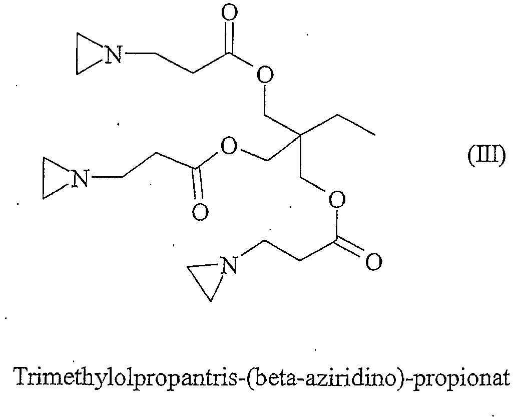

| **Lagerzeit** | **4 Wochen** | **8 Wochen** | **12 Wochen** |
|---|---|---|---|
| Probe | Viskosität [mPa·s]/ Wasserverdünnbarkeit | Viskosität [mPa·s]/ Wasserverdünnbarkeit | Viskosität [mPa·s]/ Wasserverdünnbarkeit |
| **A1, RT** | 190 / 1 | 210 / 1 | 260 / 1 |
| **A1, 50 °C** | 320 / 1 | 420 / 1 | 500 / 2 |
| **A2, RT** | 260 / 1 | 270/ 1 | 270 / 1 |
| **A2, 50 °C** | 300 / 1 | 300 / 1 | 300 / 1 |
| **B, RT** | 580 / 3 | n.m. | n.m. |
| **B, 50 °C** | 1950 / 5 | n.m. | n.m. |
| **C, RT** | 320 / 1 | 510 / 2 | 610 / 3 |
| **C, 50 °C** | 1360 / 4 | n.m. | n.m. |
| **D, RT** | 530 / 3 | 590 / 3 | 650 / 3 |
| **D, 50 °C** | 1020 / 4 | 1450 / 4 | 1620 / 4 |
| **E, RT** | 410 / 3 | 440 / 3 | 440 / 3 |
| **R, 50°C** | 800 / 4 | 1480 / 4 | n.m. |

| | | | |
|---|---|---|---|
| n.m.: nicht messbar (Probe verdickt) | | | |

Die erfindungsgemäßen Zusammensetzungen weisen eine größere Lagerstabilität, eine geringere Viskosität und bessere Wasserverdünnbarkeit auf.

### b) Vergilbungsneigung

Polyfunktionelle Aziridinverbindungen werden als Komponenten in Formulierungen eingesetzt, die in der Lederbehandlung bzw. -veredelung verwendet werden. Eine unerwünschte Winkung der Aziridinhärter ist die stärkere Vergilbung der behandelten Leder. Die neue Formulierung mit 1,4-Diazabicyclo[2.2.2]octan zeigt gegenüber dem Stand der Technik eine deutlich geringere Vergilbungsneigung.

Zur Bestimmung der Vergilbungsneigung wurde zunächst eine standardisierte Oberfläche (weiß grundierte Alufolie) hergestellt und diese dann mit einer typischen Oberflächenveredelungs-Rezeptur (Ansatz Top) behandelt. Nach definierter Lagerung wird die Vergilbung gemessen.

**Ansatz für die Grundierung der Alufolie:**

| | |
|---|---|
| Astacin Finish PF | 400 Tl |
| Leptonvcreiß N | 100 Tl |
| VE-Wasser | 200 Tl, |

spritzen bis zur Deckung (15g/DIN A4), dann trocknen bei 80°C.

**Ansatz der Lederbehandlungsformulierungen (Ansatz Top):**

| | | **1** | **2** | **3** |
|---|---|---|---|---|
| | | 500 | 500 | 500 |
| VE-Wasser | | 460 | 450 | 450 |
| Verdicker 6 | | 40 | 40 | 40 |
| Probe E (Stand der Technik) | | | 10 | |
| Probe A2 (erfindungsgemäß) | | | | 10 |

Verdicker 6 = handelsüblicher Polyurethan-Assoziativ-Verdicker, beispielsweise: 50 : 50-Mischung aus Collacral^{®} PU85 und Solvenon^{®}DPM.

### Auftragen auf weiß grundierter Alufolie

Auftragsmenge: 20 g/Qfs = 20 g/m²
Tests: Wärmebelagerung und Vergilbungsmenge mit BCS-Win Programm

Grundlage für die Messung ist das Cielab-Farbmetriksystem, welches in der DIN 5033 (Blatt 1-9) beschrieben ist. Die Messung erfolgt insbesondere zur "Farbmetrischen Bestimmung von Farbabständen bei Körperfarben nach der CIELab-Formel" gemäß DIN 6174 mit
dE = Gesamtfarbabstand und
db = Farbabstand in Richtung der Gelbachse.

| | | **1** | **2** | **3** |
|---|---|---|---|---|
| 4h, 140 °C | dE | 0,42 | 0,42 | 0,24 |
| | db | -0,09 | 0,34 | 0,1 |
| 6d, 100 °C | dE | 0,31 | 1,7 | 0,72 |
| | db | 0,11 | 1,58 | 0,66 |

Mit der erfindungsgemäßen Formulierung A2 kann die Wärmevergilbung (dE Gesamtfarbveränderung, db Gelbanteil nach CIELab) auf weniger als halb so große Werte wie bei Formulierungen des Standes der Technik verringert werden.

### c) Vernetzerwirkung

Zur Bestimmung der Vernetzerwirkung wurden übliche Bindermischungen zur Lederbehandlung mit den normalen Mengen Härter versetzt, Lederstücke zugerichtet und bezüglich Härtung, speziell Nassreibechtheit, geprüft:

### Zurichtung auf ungefärbtes Rindboxleder

**Ansätze**

| Versuchsnummer | 1a | 1b | |
|---|---|---|---|
| Wasser | 400 | 400 | |
| Probe E (Stand der Technik) | 6 | | |
| Probe A1 (erfindungsgemäß) | | 6 | |
| Lepton Schwarz N | 100 | 100 | Anm.: Pigmentpräparation aus Ruß gemäß DE 41 42 240 |
| Corial Mikrobinder AM | 300 | 300 | Anm.: Acrylatbinder gemäß DE 33 44 354 |

Wasser und die "Probe" wurden jeweils vorgemischt und zu einer Mischung aus Mikrobinder AM und Lepton Schwarz geben, dann erfolgten zwei Kreuz nass spritzen (Auftrag mit Spritzpistole, entspricht viermaligem Auftrag (zweimal von oben nach unten und zweimal von links nach rechts)) (ca.15g/DIN A4), dann ein Trocknen im Trockenkanal 3X. Anschließend wurden die Proben bei 70 °C / 50 bar für 3 Sekunden gebügelt. Anschließend erfolgte ein zweiter Spritzauftrag mit anschließendem Trocknen (ohne Bügeln).

Die Proben wurden bei Zimmertemperatur 2h gelagert und dann sofort bezüglich Nassreibechtheit untersucht (Grenzwert = 60 Reibtouren ohne Beschädigung)

| | | |
|---|---|---|
| Nassreibechtheit 60x Beschädigung | 0 | 0 |

Bewertung/Beschädigung: 0 = keine, g = gering, d = deutlich, s = stark, ss = sehr stark Mit der erfindungsgemäßen Formulierung A1 wird die gleiche anwendungstechnische Winkung erzielt, wie beim Stand der Technik (trotz höherem Stabilisatorgehalt). Der erhöhte Stabilisatorgehalt führt nicht zu einer verminderten oder verzögerten Wirkung.

## Patentansprüche

1. Verfahren zur Herstellung einer Zusammensetzung enthaltend mindestens eine polyfunktionelle Aziridinverbindung, 1,4-Diazabicyclo[2.2.2]octan und ein polares, nicht reaktives Lösemittel ausgewählt aus Diacetonalkohol oder N-Methyl-pyrrolidinon, wobei der Gehalt an 1,4-Diazabicyclo[2.2.2]octan 0,1 bis 10 Gew.-%, jeweils bezogen auf die Zusammensetzung, beträgt, **gekennzeichnet durch** die folgenden Verfahrensschritte:
(a) Bereitstellung einer Mischung aus gegebenenfalls substituiertem Ethylenimin und 1,4-Diazabicyclo[2.2.2]octan;
(b) Zugabe mindestens eines mit α,β-ungesättigten Carbonsäuren veresterten mehrwertigen Alkohols und/oder mindestens eines Polyisocyanates.

2. Verfahren nach Anspruch 1, **dadurch gekennzeichnet, dass** der Alkohol ausgewählt ist aus der Gruppe bestehend aus Trimethylolpropan, Neopentylglykol, Glycerin, Pentaerythrit, 4,4'-Isopropylidendiphenol und 4,4'-Methylendiphenol.

3. Verfahren nach Anspruch 1 oder 2, **dadurch gekennzeichnet, dass** die α,β-ungesättigte Carbonsäure ausgewählt ist aus der Gruppe bestehend aus Acryl- und Methacrylsäure, Crotonsäure und Zimtsäure.

4. Verfahren nach einem der Ansprüche 1 bis 3, **dadurch gekennzeichnet, dass** das Polyisocyanat ausgewählt ist aus der Gruppe bestehend aus Hexamethylendiisocyanat, 4,4'-Methylen-bis-(phenylisocyanat) und 1,3,5-Tris(ω-hexamethylenisocyanato)-biuret.

5. Verwendung einer Zusammensetzung, enthaltend mindestens eine polyfunktionelle Aziridinverbindung, 1,4-Diazabicyclo[2.2.2]octan und ein polares, nicht reaktives Lösemittel, ausgewählt aus Diacetonalkohol oder N-Methyl-pyrrolidinon, mit einem Gehalt an 1,4-Diazabicyclo[2.2.2]octan von 0,1 bis 10 Gew.-%, jeweils bezogen auf die Zusammensetzung, als Härterkomponente für Formulierungen im Bereich der Lederbehandlung, der Beschichtungen, des Textildrucks und der Lacke.

6. Lederbehandlungsmittel, Beschichtungsmittel, Textildruckmittel oder Lacke, enthaltend eine Zusammensetzung, enthaltend mindestens eine polyfunktionelle Aziridinverbindung, 1,4-Diazabicyclo[2.2.2]octan und ein polares, nicht reaktives Lösemittel, ausgewählt aus Diacetonalkohol oder N-Methyl-pyrrolidinon, mit einem Gehalt an 1,4-Diazabicyclo[2.2.2]octan von 0,1 bis 10 Gew.-%, jeweils bezogen auf die Zusammensetzung, als Härterkomponente.

## Claims

1. A process for the preparation of a composition comprising at least one polyfunctional aziridine compound, 1,4-diazabicyclo[2.2.2]octane and a polar, unreactive solvent selected from diacetone alcohol or N-methylpyrrolidone, the content of 1,4-diazabicyclo[2.2.2]octane being from 0.1 to 10% by weight, based in each case on the composition, comprising the following process steps:
(a) provision of a mixture of unsubstituted or substituted ethylenimine and 1,4-diazabicyclo[2.2.2]octane;
(b)addition of at least one polyhydric alcohol esterified with α,β-unsaturated carboxylic acids and/or of at least one polyisocyanate.

2. The process according to claim 1, wherein the alcohol is selected from the group consisting of trimethylolpropane, neopentylglycol, glycerol, pentaerythritol, 4,4'-isopropylidenediphenol and 4,4'-methylenediphenol.

3. The process according to claim 1 or 2, wherein the α,β-unsaturated carboxylic acid is selected from the group consisting of acrylic and methacrylic acid, crotonic acid and cinnamic acid.

4. The process according to any of claims 1 to 3, wherein the polyisocyanate is selected from the group consisting of hexamethylene diisocyanate, 4,4'-methylenebis(phenyl isocyanate) and 1,3,5-tris(ω-hexamethyleneisocyanato)biuret.

5. The use of a composition comprising at least one polyfunctional aziridine compound, 1,4-diazabicyclo[2.2.2]octane and a polar, unreactive solvent selected from diacetone alcohol or N-methylpyrrolidone, and a content of 1,4-diazabicyclo[2.2.2]octane of from 0.1 to 10% by weight, based in each case on the composition, as a curing component for formulations in the area of leather treatment, of coatings, of textile printing and of surface coatings.

6. A leather treatment composition, coating composition, textile printing composition or surface coating, comprising a composition comprising at least one polyfunctional aziridine compound, 1,4-diazabicyclo[2.2.2]octane and a polar, unreactive solvent selected from diacetone alcohol or N-methylpyrrolidone, and a content of 1,4-diazabicyclo[2.2.2]octane of from 0.1 to 10% by weight, based in each case on the composition, as a curing component.

## Revendications

1. Procédé de fabrication d'une composition contenant au moins un composé d'aziridine polyfonctionnel, du 1,4-diazabicyclo[2.2.2]octane et un solvant polaire non réactif choisi parmi l'alcool diacétonique ou la N-méthylpyrrolidone, la teneur en 1,4-diazabicyclo[2.2.2]octane étant de 0,1 à 10 % en poids, à chaque fois par rapport à la composition, **caractérisé par** les étapes de procédé suivantes :
(a) la préparation d'un mélange d'éthylène-imine éventuellement substituée et de 1,4-diazabicyclo[2.2.2]octane ;
(b) l'ajout d'au moins un alcool polyvalent estérifié avec des acides carboxyliques α,β-insaturés et/ou d'au moins un polyisocyanate.

2. Procédé selon la revendication 1, **caractérisé en ce que** l'alcool est choisi dans le groupe constitué par le triméthylolpropane, le néopentylglycol, la glycérine, la pentaérythrite, le 4,4'-isopropylidène-diphénol et le 4,4'-méthylène-diphénol.

3. Procédé selon la revendication 1 ou 2, **caractérisé en ce que** l'acide carboxylique α,β-insaturé est choisi dans le groupe constitué par l'acide acrylique et méthacrylique, l'acide crotonique et l'acide cinnamique.

4. Procédé selon l'une quelconque des revendications 1 à 3, **caractérisé en ce que** le polyisocyanate est choisi dans le groupe constitué par le diisocyanate d'hexaméthylène, le bis-(phénylisocyanate) de 4,4'-méthylène et le 1,3,5-tris(ω-hexaméthylène-isocyanato)-biuret.

5. Utilisation d'une composition contenant au moins un composé d'aziridine polyfonctionnel, du 1,4-diazabicyclo[2.2.2]octane et un solvant polaire non réactif choisi parmi l'alcool diacétonique ou la N-méthylpyrrolidone, ayant une teneur en 1,4-diazabicyclo[2.2.2]octane de 0,1 à 10 % en poids, à chaque fois par rapport à la composition, en tant que composant durcisseur pour des formulations dans le domaine du traitement du cuir, des revêtements, de l'impression sur textile et des vernis.

6. Agents de traitement du cuir, agents de revêtement, agents d'impression sur textile ou vernis, contenant une composition, qui contient au moins un composé d'aziridine polyfonctionnel, du 1,4-diazabicyclo[2.2.2]octane et un solvant polaire non réactif choisi parmi l'alcool diacétonique ou la N-méthylpyrrolidone, ayant une teneur en 1,4-diazabicyclo[2.2.2]octane de 0,1 à 10 % en poids, à chaque fois par rapport à la composition, en tant que composant durcisseur.
